Europäisches Patentamt

European Patent Office

Office européen des brevets

(19)

(11) Publication number: **0 174 152**
**B1**

(12) **EUROPEAN PATENT SPECIFICATION**

(45) Date of publication of patent specification: **12.07.89**

(51) Int. Cl.⁴: **A 61 F 13/00, A 61 F 13/18**

(21) Application number: **85306079.6**

(22) Date of filing: **28.08.85**

(54) Absorbent products.

(30) Priority: **01.09.84 GB 8422143**

(43) Date of publication of application:
**12.03.86 Bulletin 86/11**

(45) Publication of the grant of the patent:
**12.07.89 Bulletin 89/28**

(84) Designated Contracting States:
**BE CH DE FR GB LI NL**

(56) References cited:
**AT-B- 320 559**
**DE-A-2 146 790**
**DE-B-1 164 020**
**GB-A-1 548 865**
**GB-A-2 080 351**
**GB-A-2 089 214**

(73) Proprietor: **Smith and Nephew Associated Companies p.l.c.**
**2, Temple Place Victoria Embankment London WC2R 3BP (GB)**

(72) Inventor: **Williams, Pamela Joan**
**58A Rosemary Hill Road**
**Sutton Cold Field West Midlands (GB)**

(74) Representative: **Cole, William Gwyn Corporate Patents Department Smith and Nephew Research Limited Gilston Park Harlow Essex CM20 2RQ (GB)**

Courier Press, Leamington Spa, England.

# EP 0 174 152 B1

## Description

The present invention relates to an hygienic absorbent pad which comprises a layer of liquid pervious material adapted to promote the spread of fluid within the pad and processes for its manufacture.

It is well known that in use the available absorbent capacity of hygienic absorbent pads such as sanitary towels and alike absorbent pads is rarely fully utilised. This under utilisation of the absorbent capacity occurs because hygienic absorbent pads such as sanitary towels normally have an elongate shape and the spread of fluid within such towels is often confined to the central area of the towel. It is known to provide the absorbent-core of a sanitary towel with a tissue wadding insert in order to promote lateral spread of fluid within the towel and also to provide reinforcement for the absorbent material used in the towel. Such wadding insert, however, undesirably increases the bulk and stiffness of the sanitary towel and also tends to promote the spread of fluid at a similar rate in both the longitudinal and width directions of the towel with the result that the absorbed fluid spreads to the side edges of the towel adjacent to the exuding area before it can spread to the ends of the towel.

United Kingdom Application No. 2017509B discloses an absorbent pad which includes at least one layer of folded or pleated crepe paper which absorbs moisture more readily in the direction of the folds or pleats than the transverse direction.

United Kingdom Application No. 2080351A discloses an absorbent pad comprising at least two layers of absorbent material having between them a corrugated diffuser layer comprising a plurality of plies of creped tissue which spreads fluid preferentially in the longitudinal direction of the corrugations. The absorbent pads of both these patents have a layer which promotes the spread of fluid in a preferential direction within the pads and which therefore improves the utilisation of the absorbent capacity of such pads.

The spreading layer of the absorbent pads disclosed in these patents, however, comprises longitudinal pleated, folded or corrugated crepe paper or tissue. The use of such a layer can undesirably increase the bulk and stiffness of the absorbent pad.

United Kingdom Patent No. 1548865 discloses a plastic net, suitable for use as cover layer on a sanitary towel, which comprises a plurality of ribs in one direction interconnected by a plurality of fibrillated strands. It has been found, however, that a cover layer of such a plastics net on a towel in use, although it allows passage of fluid to the absorbent core thereof, does not promote the spread of fluid in one direction within the towel.

It has now been found that an hygienic absorbent pad such as a sanitary towel can be provided which has a layer which promotes the spread of fluid in a preferred direction, but does not substantially increase the bulk or stiffness of the absorbent pad.

Accordingly the present invention provides an hygienic absorbent pad which comprises an absorbent core and a layer of liquid pervious material adapted to promote the spread of fluid in one direction within the pad characterised in that the layer of material is a plastics net which comprises a plurality of ribs in one direction interconnected by a plurality of fibrillated strands in a transverse direction and said layer, is positioned within the absorbent core or adjacent to the non-body facing surface of the absorbent core, the direction of the ribs defining the one direction.

The term "net" when used herein means a net in which the strands or ribs and junctures are formed integrally. Fibrillated strands are strands which have been wholly or partly split into fibrils or small fibres. Such strands will be thinner than the ribs of the net.

The hygienic absorbent pad of the invention can be a sanitary towel or alike absorbent pad which has a elongate shape such as an incontinence pad, maternity pad, or diaper. It is preferred however, that the hygienic absorbent pad is a sanitary towel.

The invention will now be described in relation to a sanitary towel of the invention but is not intended that the invention so described should be limited to such a hygienic absorbent pad.

It has been found that the layer of plastics net used in the invention promotes the spread of fluid within a sanitary towel of the invention in the direction of its ribs.

In a preferred sanitary towel of the invention the ribs of the layer of plastics net lie in the length direction of the sanitary towel. In such an arrangement the layer of plastic net advantageously promotes the spread of fluid within the towel in the length direction and towards the ends of the sanitary towel, thereby increasing the utilisation of the capacity of the sanitary towel in use.

Suitable plastics nets for use in the invention include the plastics nets disclosed in British Patent No. 1,548,865. These plastics nets comprise substantially straight parallel longitudinal ribs interconnected by a plurality of fibrillated strands. A net of this type has porosity, strength and softness such that when the net is used as a spreading layer within the towel, it will allow transmission of fluid therethrough and also advantageously provide reinforcement for the absorbent material of the towel without unduly increasing the stiffness of the sanitary towel.

The net used in the invention will normally comprise a thermoplastic polymer which is relatively non-water absorbent such as a polyolefine or polyamide.

The net used in the invention preferably comprises a blend of incompatible polymers. Suitable blends comprise a major proportion of a polyolefine such as polypropylene, high density polyethylene or ethylene-propylene copolymers and a minor proportion of an incompatible polymer such as polystyrene

2

for example high impact polystyrene or polyamide.

Favoured nets for use in the invention comprise a blend of high density polyethylene and 5 to 20% by weight of high impact polystyrene.

The ribs and interconnecting fibrillated strands define the holes or apertures in the plastics net and therefore the size of these holes or apertures will depend to some extent on density of ribs and fibrillated strands in the net. The density of ribs and strands in the net, however, should be sufficiently high to allow the net in use to spread fluid within the sanitary towel in the direction of the ribs but sufficiently low to allow the passage of fluid through the net.

The plastics net used in the invention can suitably have 4 to 20 ribs/cm and can preferably have 5 to 15 ribs/cm. Similarly the plastics net can suitably have 8 to 50 fibrillated strands/cm and can preferably have 10 to 40 fibrillated strands/cm.

Suitably the plastics nets for use in the invention have a weight per unit area of 2 to $30g/m^2$ desirably have a weight per unit area of 2 to $20g/m^2$, and preferably have a weight unit area of 4 to $12g/m^2$.

Suitably the plastic nets for use in the invention have a thickness of 0.03mm to 0.150mm and preferably have a thickness of 0.04mm to 0.10mm. Such nets when used as a net spreading layer in a sanitary towel of the invention therefore have a thickness which will not significantly increase the bulk of the sanitary towel.

Apt plastics nets for use in the invention include those available as net 909 (Trade mark) grades A4, A7, H8C and DHM (available from Smith and Nephew Plastics Ltd.). Such apt nets comprise a blend of high density polyethylene and 8 to 10% by weight of high impact polystyrene, and have a weight per unit area of 5 to $11g/m^2$, a thickness of 0.40 to 0.120mm. A favoured net of this type is net 909 grade H8C which has a weight per unit area of $9g/m^2$, a thickness of 0.06mm and 11/cm longitudinal ribs interconnected by approximately 15/cm fibrillated strands.

In order to promote the spread of fluid in a preferred direction within the towel, the layer of plastics net should be positioned adjacent to an upper layer of absorbent material. The layer of plastics net therefore will not normally be effective as a spreader layer when positioned adjacent to the body facing surface of the absorbent core of the towel.

The layer of plastics net is preferably positioned within the absorbent core of the sanitary towel.

In favoured sanitary towels of the invention the absorbent core comprises two layers of absorbent material and the layer of net is positioned between the two layers.

The sanitary towel of the invention however, can comprise more than one layer of plastic net positioned within the absorbent core of the towel. Such net layers can be in a contiguous relationship with each other or preferably can be positoned at spaced intervals within the thickness of the absorbent core.

The direction of the longitudinal ribs of such net layers can be in the same direction for example in the length direction of the sanitary towel to promote preferential spread of fluid to the ends thereof. However, the direction of the longitudinal ribs of such net layers can lie in different directions in order to promote the preferential spread of fluid to different parts of the absorbent core.

The absorbent materials used in the invention include those which are conventionally used for sanitary towel manufacture. Suitable materials include cellulosic materials such as comminuted/fluffed wood pulp, carded cotton webs, regenerated cellulose fibres, and tissue paper and super absorbent materials such as grafted cellulose super absorbents, polymeric super absorbents and mixtures of such materials.

The sanitary towel of the invention can have a fluid impervious barrier sheet covering the non-body facing surface of absorbent core of the towel and preferentially also the longitudinal side edges of the absorbent core.

In another preferred sanitary towel of the invention the layer of plastic net is positioned in contiguous relationship to the absorbent core and between the absorbent core and the fluid impervious barrier sheet. In this arrangement the layer of plastics net promotes the spread of fluid in a preferred direction in the layer of absorbent material adjacent to the net on the non-body contacting face of the sanitary towel.

The fluid impervious sheet used in the invention is suitably any inert physiologically compatible plastics sheet which includes sheets made from polyethylene, polypropylene, polyamides, polyvinyl chloride, polyvinyl acetate, insoluble polyvinyl alcohol and polyurethane. Preferably the fluid impervious sheet is a polyethylene or polypropylene sheet. Suitably the sheet is from 0.005 to 0.06mm thick and preferably is from 0.010mm to 0.025mm thick.

The layer of plastic net used in the invention should extend over the central area of the sanitary towel to enable the layer to promote the spread within the towel of fluid exuding onto the body contacting side of the sanitary towel in use. Preferably the layer of plastics net extends in a lengthwise direction to both ends of the towel to enable the layer to promote the spread of fluid preferentially in the direction of such ends. Similarly it is preferable that the width of the layer of plastics net is less than the width of the absorbent core in order to inhibit the spread of fluid in the width direction of the towel.

In favoured sanitary towels of the invention the layer of plastics net is in the form of a strip which extends in the length directions of the towel to both ends thereof and which has a width which is less than that of the absorbent core.

A sanitary towel of the invention will normally comprise a fluid pervious layer which covers the body contacting face of the towel. Preferably the fluid pervious cover layer extends around the longitudinal sides and the non-body contacting face of the sanitary towel where it can conveniently be sealed in an overlapping relationship, for example by application of an adhesive to form a wrapper for the sanitary towel.

The fluid pervious cover layer can be any of the cover layers conventionally used on sanitary towels. Suitable cover layers include spun bonded, thermally bonded and resin bonded non-woven fabrics, and plastics nets.

Favoured bonded non-woven fabrics for use as a cover layer include thermally-bonded and spun-bonded polypropylene.

Favoured plastics net for use as a cover layer include the nets hereinbefore disclosed in relation to the net spreading layer used in the invention.

Optionally the sanitary towels of the present invention will also have placed between the core and the wrapper a fleece material which provides the towel with extra softness and comfort as well as contributing to the effectiveness of the wrapper to prevent cover wetness. Typically the fleece material will be positioned across the body contacting side of the device beneath the fluid permeable cover or it may extend so as to also lie between the fluid impervious sheet and the core or between the fluid impervious sheet and the wrapper along the side of the core. Suitably the fleece will be made from polyester, polypropylene, polyamide, cotton or regenerated cellulose or a mixture of these fibres. A preferred fleece material is formed from a carded viscose rayon web.

In another aspect the invention provides a process for the manufacture of a hygienic absorbent pad of the invention which comprises providing a layer of liquid pervious material within the pad adapted to promote the spread of fluid therein in one direction characterised in that the layer of material is a plastics net which comprises a plurality of ribs in one direction interconnected by a plurality of fibrillated strands in a transverse direction and said layer is positioned within or adjacent to the non-body facing surface of the absorbent core, the direction of the ribs defining the one direction.

The hygienic absorbent pad made by the process of the invention can be a sanitary towel or alike absorbent pad which has an elongate shape such as an incontinence pad, maternity pad or diaper. It is preferred, however, that the hygienic absorbent pad is a sanitary towel.

The plastics net used in the process of the invention can suitably be the plastics net hereinbefore mentioned in relation to the sanitary towels of the invention. Such nets can be conveniently made by the methods disclosed in British Patent No. 1,548,865.

In the process of the invention the component layers used for forming the towel, for example layers in the form of strips, can be combined in separate steps to form the sanitary towel. Materials for the component layers are suitably the same as hereinbefore described in relation to the sanitary towel of the invention.

A process of forming a preferred sanitary towel of the invention comprises the step of providing a layer of plastic net between two layers of absorbent material.

The combined layers of such a process can conveniently be in the form of a strip which can be cut into suitable lengths by a conventional means to provide elongate absorbent cores of individual sanitary towels. A series of such elongate absorbent cores can then be placed onto a wider strip fluid previous cover fabric which can then be folded around individual cores and sealed by application of adhesive to the overlapping longitudinal edges of the fabric to form a wrapper for the core. The wrapper strip can then be heat sealed and cut adjacent to both ends of individual absorbent cores in a conventional manner to form sanitary towels of the invention.

Other components of towels such as a fluid impervious barrier sheet or a fleece may be placed on the appropriate face of the absorbent core prior to the application of the wrapper layer.

A process of forming a less preferred sanitary towel of the invention comprises the step of providing a layer of plastic net in a contiguous relationship to and between the absorbent core and a fluid impervious barrier sheet. The absorbent core can then be provided with a wrapper and formed into a sanitary towel and hereinbefore described.

The invention will now be illustrated by reference to the following drawings.

Figure 1 is a diagrammatic plan view of a sanitary towel of the invention showing the position of the net layer within the towel.

Figure 2 is an enlarged cross-sectional view of Figure 1.

Figure 3 is a cross-sectional view of another sanitary towel of the invention.

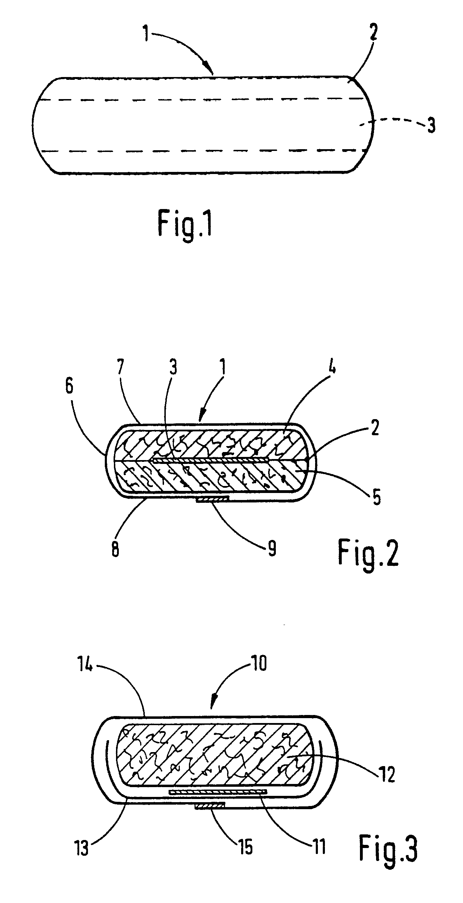

Figure 1 and 2 shows a sanitary towel (1) which comprises elongate absorbent core (2) containing a layer of plastics net (3) comprising longitudinal ribs (not shown) interconnected by fibrillated strands (not shown). The absorbent core (2) of the sanitary towel (1) consists of two layers (4, 5) of absorbent material and the layer of plastics net is positioned between these absorbent layers (4, 5). The sanitary towel has a wrapper (6) of fluid pervious material which covers the body-contacting face (7) of the towel and also extends around the longitudinal side edges and non-body contacting face (8) of the towel. The overlapping edge portions of the wrapper (6) are sealed by adhesive (9). As shown in Figure 1 the layer of plastics net (3) is in the form of a strip which extends substantially to the ends of the towel but is narrower than the width of the towel.

The sanitary towel (10) shown in Figure 3 has a strip layer of plastics net (11), similar to that used in the sanitary towel of Figures 1 and 2, which is positioned in contiguous relationship to the absorbent core (12) and between absorbent core (12) and fluid impervious barrier sheet (13) covering the non-body contacting face and longitudinal sides of the core.

Sanitary towel (10) has a wrapper (14) which surrounds the absorbent core (12) and is sealed at its overlapping edges by means of adhesive (15).

The longitudinal ribs (not shown) of the strip layers of plastic net used in each of the two sanitary towel embodiments shown in Figure 1 to 3 are in the length direction of the sanitary towel. Any body fluid which penetrates to the strip layer of plastics net within such sanitary towels will therefore spread preferentially in the length direction of the towels thereby increasing the utilisation of the absorbent capacity of the towels.

Example

A 40mm wide strip of plastics net having parallel straight longitudinal ribs interconnected by fibrillated strands (reference H8C available from Smith & Nephew Plastics) was fed between two 65mm wide batts of fluffed wood pulp onto 70mm wide strip of viscose rayon fleece (weight per unit area 51g/m$^2$) and combined to form a strip which was cut into individual absorbent strips 216mm long.

Absorbent strips containing the net layer were then fed longitudinally onto a 200mm wide strip of thermally bonded polypropylene cover fabric (Finntex) which was folded around the sides and over the top face of the absorbent strips and sealed by application of an adhesive to the overlapping portions of fabric strip. The folded cover fabric containing the absorbent strips was heat sealed adjacent to the ends of the absorbent strips and cut into suitable lengths to form sanitary towels of the invention.

The absorbent core of the sanitary towels contained 9.5g of fluffed wood pulp. The longitudinal ribs of the integral net within the absorbent core were in the length direction of the sanitary towel.

Spreading Test

In order to demonstrate the fluid spreading action of the net within the towel, sanitary towels made according to the example and comparison sanitary towels without a net layer, were tested by the following procedure:

1. The sanitary towel was folded lengthways and a 1 Kg weight was placed on the folded sanitary towel for 30 sec and then removed. The weight on the folded towel simulates the distortion pressures found in use.

2. The sanitary towel was then unfolded and 4 ml of 1% saline solution containing a lissamine green dye were applied by means of a dropper at an even rate over a period of 4 minutes onto the centre of the towel.

3. After a lapse of 3 mins a 2 Kg weight measuring 145mm × 60mm was placed on the towel for 1 minutes.

4. The towel was then opened up at the net layer position and the length of fluid spread in the adjacent top batt recorded.

Four samples of each sanitary towel were tested and the following results were obtained:

Length of fluid spread (mm)

| Sample No. | 1 | 2 | 3 | 4 | Average |
|---|---|---|---|---|---|
| Sanitary towel with net layer | 150 | 90 | 95 | 115 | 112.5 |
| Sanitary towel without net layer | 95 | 75 | 70 | 65 | 76.25 |

The results showed that the net layer of sanitary towels of the invention substantially increased (average increase 47.5%) the spread of fluid in the length direction of these sanitary towels when compared to that of a sanitary towel without such a net layer. It was also found in the test that the net layer limited the spread of fluid in the width direction of the pad.

**Claims**

1. An hygienic absorbent pad which comprises an absorbent core (2) and a layer of liquid pervious material adapted to promote the spread of fluid in one direction within the absorbent pad characterised in that the layer of liquid pervious material is a plastics net (3) which comprises a plurality of ribs in one direction interconnected by a plurality of fibrillated strands in a transverse direction and said layer is positioned within the absorbent core or adjacent to the non-body facing surface of the absorbent core, the direction of ribs defining the one direction.

2. An hygienic absorbent pad as claimed in claim 1 which is a sanitary towel.

3. An hygienic absorbent pad as claimed in either of claims 1 or 2 in which the layer of plastics net is positioned within the absorbent core of the pad.

4. An hygienic absorbent pad as claimed in claim 3 in which the absorbent core comprises two layers of absorbent material and the layer of plastics net is positioned between the two layers.

5. An hygienic absorbent pad as claimed in either of claims 1 or 2 in which the pad comprises a fluid impervious barrier sheet covering the non-body facing surface of the absorbent core and the layer of plastics net is positioned in a contiguous relationship to the absorbent core and between the absorbent core and the barrier sheet.

6. An hygienic absorbent pad as claimed in any of claims 1 to 5 in which the layer of plastics net has a width which is less than that of the absorbent core of the pad.

7. An hygienic absorbent pad as claimed in any of claims 1 to 6 in which the layer of plastics net extends to both ends of the pad.

8. An hygienic absorbent pad as claimed in any of claims 1 to 7 in which the layer of plastics net comprises a blend of polyolefine and an incompatible polymer.

9. An hygienic absorbent pad as claimed in any of claims 1 to 8 in which the layer of plastics net has a thickness of 0.04mm to 0.10mm.

10. A process for the manufacture of an hygienic absorbent pad comprising an absorbent core as claimed in any of claims 1 to 9 which comprises providing a layer of liquid pervious material within the pad adapted to promote the spread of fluid therein in one direction characterised in that the layer of material is a plastics net which comprises a plurality of ribs in one direction interconnected by a plurality of fibrillated strands in a transverse direction and said layer is positioned within or adjacent to the non-body facing surface of the absorbent core, the direction of the ribs defining the one direction.

**Patentansprüche**

1. Hygienisches absorbierendes Kissen, das einen absorbierenden Kern (2) und eine Schicht aus flüssigkeitsdurcblässigem Material, welches die Verteilung der Flüssigkeit in eine Richtung begünstigt, aufweist, dadurch gekennzeichnet, daß die Schicht aus flüssigkeitsdurchlässigem Material ein Kunststoffnetz (3) ist, welches eine Vielzahl von Rippen in einer Richtung aufweist, die durch eine Vielzahl von feinfaserigen Fäden in Querrichtung verbunden sind, daß diese Schicht entweder innerhalb des absorbierenden Kerns oder an der vom Körper abgewandten Oberfläche des absorbierenden Kerns angeordnet ist und daß die Richtung der Rippen die eine Richtung definiert.

2. Hygienisches absorbierendes Kissen nach Anspruch 1, welches eine Damenbinde ist.

3. Hygienisches absorbierendes Kissen nach Anspruch 1 oder 2, bei welchem die aus dem Kunststoffnetz gebildete Schicht innerhalb des absorbierenden Kerns angeordnet ist.

4. Hygienisches absorbierendes Kissen nach Anspruch 3, bei welchem der absorbierende Kern zwei Schichten aus absorbierendem Material aufweist und die aus dem Kunststoffnetz gebildete Schicht zwischen diesen beiden Schichten angeordnet ist.

5. Hygienisches absorbierendes Kissen nach Anspruch 1 oder 2, bei welchem das Kissen eine flüssigkeitsundurchlässige Barriere-Lage aufweist, die die vom Körper abgewandte Oberfläche des absorbierenden Kerns bedeckt und die aus dem Kunststoffnetz gebildete Schicht am absorbierenden Kern unmittelbar anliegend und zwischen diesem und der Barriere-Lage angeordnet ist.

6. Hygienisches absorbierendes Kissen nach einem der Ansprüche 1 bis 5, bei welchem die aus dem Kunststoffnetz gebildete Schicht eine kleinere Breite als die des absorbierenden Kerns hat.

7. Hygienisches absorbierendes Kissen nach einem der Ansprüche 1 bis 6, bei welchem die aus dem Kunststoffnetz gebildete Schicht sich bis zu beiden Enden des Kissens erstreckt.

8. Hygienisches absorbierendes Kissen nach einem der Ansprüche 1 bis 7, bei welchem die aus dem Kunststoffnetz gebildete Schicht eine Mischung aus Polyolefin und einem nicht kompatiblen Polymer aufweist.

9. Hygienisches absorbierendes Kissen nach einem der Ansprüche 1 bis 8, bei welchem die aus dem Kunststoffnetz gebildete Schicht eine Dicke zwischen 0,04 mm bis 0,1 mm hat.

10. Verfahren zum Herstellen eines hygienischen absorbierenden Kissens, das einen absorbierenden Kern nach einem der Ansprüche 1 bis 9 aufweist, wobei eine Schicht aus flüssigkeitsdurchlässigem Material innerhalb des Kissens vorgesehen ist, die die Verteilung der Flüssigkeit darin in eine Richtung begünstigt, dadurch gekennzeichnet, daß die Materialschicht ein Kunststoffnetz ist, das eine Vielzabl von Rippen in einer Richtung aufweist, die durch eine Vielzahl von feinfaserigen Fäden in Querrichtung verbunden sind, daß diese Schicht entweder innerhalb des absorbierenden Kerns oder an der vom Körper abgewandten Oberfläche des absorbierenden Kerns angeordnet ist und daß die Richtung der Rippen die eine Richtung definiert.

**Revendications**

1. Coussinet absorbant hygiénique qui comprend un noyau absorbant (2) et une couche de matiére perméable aux liquides adaptée pour favoriser la diffusion du fluide dans une direction particulière à l'intérieur du coussinet, caractérisé en ce que la couche de matiére perméable aux liquides est un filet (3) en matière plastique qui comprend un grand nombre de nervures dans une direction particulière interconnectèes par un grand nombre de brins fibrillés dans une direction transversale et que cette couche est disposée à l'intérieur du noyau absorbant ou au voisinage de la surface du noyau absorbant ne faisant pas face au corps, la direction des nervures définissent la direction particuliére.

2. Coussinet absorbant hygiénique suivant la revendication 1, caractérisé en ce qu'il s'agit d'une serviette hygiénique.

3. Coussinet absorbant hygiénique suivant la revendication 1 ou la revendication 2, caractérisé en ce que la couche de filet en matière plastique est disposée à l'intérieur du noyau absorbant du coussinet.

4. Coussinet absorbant hygiénique suivant la revendication 3, caractérisé en ce que le noyau absorbant comprend deux couches de matériau absorbant et que la couche de filet en matière plastique est disposèe entre les deux couches.

5. Coussinet absorbant hygiénique suivant la revendication 1 ou la revendication 2, caractérisé en ce que le coussinet comprend une feuille imperméable aux fluides formant une barrière, recouvrant la surface du noyau absorbant ne faisant pas face au corps et que la couche de filet en matière plastique est disposée contre le noyau absorbant et entre le noyau absorbant et la feuille formant une barrière.

6. Coussinet hygiénique suivant l'une quelconque des revendications 1 à 5, caractérisé en ce que la couche de filet en matière plastique a une largeur inférieure à celle du noyau absorbant du coussinet.

7. Coussinet absorbant hygiénique suivant l'une quelconque des revendications 1 à 6, caractérisé en ce que la couche de filet en matière plastique se prolonge jusqu'aux deux extrémités du coussinet.

8. Coussinet absorbant hygiénique suivant l'une quelconque des revendications 1 à 7, caractérisé en ce que la couche de filet en matière plastique comprend un mélange de polyoléfine et d'un polymère incompatible.

9. Coussinet absorbant hygiénique suivant l'une quelconque des revendications 1 à 8, caractérisé en ce que la couche de filet en matière plastique a une epaisseur de 0,04 mm à 0,10 mm.

10. Procédé de fabrication d'un coussinet absorbant hygiénique comprenant un noyau absorbant suivant l'une quelconque des revendications 1 à 9, caractérisé en ce qu'il comprend la fourniture d'une couche de matière perméable aux liquides à l'intérieur du coussinet adaptée pour favoriser la diffusion du fluide dans une direction particulière à l'intérieur du coussinet, caracterise en ce que la couche de matière est un filet en matière plastique qui comprend un grand nombre de nervures dans une direction particulière interconnectées par un grand nombre de brins fibrillés dans une direction transversale et que cette couche est disposée à l'intérieur ou au voisinage de la surface du noyau absorbant ne faisant pas face au corps, la direction des nervures définissant la direction particulière.

Fig.1

Fig.2

Fig.3